# FASCICULE DE BREVET EUROPEEN

(11) **EP 0 719 328 B1**
(45) Date de publication et mention de la délivrance du brevet: **11.10.2000**
(21) Numéro de dépôt: 94915598.0
(22) Date de dépôt: 09.05.1994
(51) Int. Cl.: C12N 15/12, A61K 48/00

(54) **GENE GRB3-3, SES VARIANTS ET LEURS UTILISATIONS**
GEN GRB3-3, VARIANTE UND IHRE VERWENDUNGEN
GRB3-3 GENE, VARIANTS AND USES THEREOF

(30) Priorité: 15.09.1993 FR 9310971
(43) Date de publication de la demande: 03.07.1996
(73) Titulaire: Aventis Pharma S.A., 92160 Antony (FR)
(72) Inventeur: SCHWEIGHOFFER, Fabien, F-94300 Vincennes (FR); TOCQUE, Bruno, F-92400 Courbevoie (FR)
(74) Mandataire: Becker, Philippe
(86) Numéro de dépôt international: FR9400542
(87) Numéro de publication internationale: WO9507981

(56) Documents cités:
- WO-A-94/07913
- SCIENCE vol. 264, no. 5161 , 13 Mai 1994 , LANCASTER, PA US pages 971 - 974 FATH, I. ET AL. 'Cloning of a Grb2 isoform with apoptotic properties'
- NATURE. vol. 363 , 6 Mai 1993 , LONDON GB pages 45 - 51 EGAN, S.E. ET AL. 'Association of SOS Ras exchange protein with Grb2 is implicated in tyrosine kinase signal transduction and transformation' cité dans la demande
- CELL. vol. 70 , 1992 , CAMBRIDGE, NA US pages 431 - 442 LOWENSTEIN, E.J. ET AL. 'The SH2 and SH3 domain-containing protein GRB2 links receptor tyrosine kinases to ras signaling' cité dans la demande
- SCIENCE vol. 259, no. 5094 , 22 Janvier 1993 , LANCASTER, PA US pages 525 - 528 DUCHESNE, M. ET AL. 'Identification of the SH3 domain of GAP as an essential sequence for Ras-GAP-mediated signaling'
- MOLECULAR AND CELLULAR BIOLOGY vol. 13 , 1993 pages 5500 - 5512 SUEN, K. ET AL. 'Molecular cloning of the mouse Grb2 gene : differential interaction of the Grb2 adaptor protein with epidermal growth factor and nerve growth factor receptor' cité dans la demande
- PROCEEDINGS OF THE NATIONAL ACADEMY OF SCIENCES OF USA. vol. 89 , 1992 , WASHINGTON US pages 9015 - 9019 MATUOKA, K. ET AL.

## Description

La présente invention concerne un nouveau gêne, désigné Grb3-3, ses variants, et leurs utilisations, notamment en thérapie génique anti-cancéreuse.

Différents gènes, appelés oncogènes et gènes suppresseurs, sont impliqués dans le contrôle de la division cellulaire. Parmi ceux-ci, les gènes ras, et leurs produits généralement désignés protéines p21, jouent un rôle clé dans le contrôle de la prolifération cellulaire chez tous les organismes eucaryotes où ils ont été recherchés. Notamment, il a été montré que certaines modifications spécifiques de ces protéines leur font perdre leur contrôle normal et les conduisent à devenir oncogénique. Ainsi, un grand nombre de tumeurs humaines a été associé à la présence de gènes ras modifiés. De même, une surexpression de ces protéines p21 peut conduire à un dérèglement de la prolifération cellulaire. La compréhension du rôle exact de ces protéines p21 dans les cellules, de leur mode de fonctionnement et de leurs caractéristiques constitue donc un enjeu majeur pour la compréhension et l'approche thérapeutique de la cancérogénèse.

Différents facteurs impliqués dans la voie de signalisation ras dépendante ont été identifiés. Parmi ceux-ci figure le gène Grb2, qui code pour une protéine de 23-25 kDa ayant une structure SH3-SH2-SH3 (Lowenstein et al., Cell 70 (1992) 431; Matuoka et al., PNAS 89 (1992) 9015). Le produit du gène Grb2 semble interagir avec les protéines tyrosine phosphorylées, par son domaine SH2, et avec un facteur d'échange du GDP de la classe SOS, par son domaine SH3 (Egan et al., Nature 363 (1993) 45). Il serait ainsi l'un des composants de l'activité transformante du produit du gène ras. La présente invention découle de la mise en évidence, du clonage et de la caractérisation d'un isoforme du gène Grb2, désigné Grb3-3, possédant une délétion dans le domaine SH2. Ce gène est exprimé dans les tissus adultes : l'ARNm correspondant est présent sous une bande unique de 1,5 kb, et est traduit en une protéine de 19 kDa. En raison de sa délétion dans le domaine SH2, le produit du gène Grb3-3 n'est plus capable d'interagir avec les protéines tyrosine phosphorylées (récepteur EGF phosphorylé), mais il conserve la capacité d'interagir avec les domaines riche en proline des protéines SOS. En raison de sa délétion, le produit du gène Grb3-3 est ainsi capable de s'opposer aux effets cellulaires du produit du gène Grb2. Le transfert de ce gène in vivo, ou de variants de celui-ci, y compris de séquences antisens, permet donc d'interférer avec les processus de prolifération, de différenciation et/ou de mort cellulaire.

Un premier objet de l'invention concerne donc une séquence nucléotidique comprenant tout ou partie du gêne Grb3-3 (séquence SEQ ID n° 1).

Un autre objet de l'invention concerne une séquence nucléotidique dérivée de la séquence SEQ ID n° 1 et capable d'inhiber au moins en partie l'expression de la protéine Grb2 ou Grb3-3. En particulier, l'invention concerne les séquences antisens, dont l'expression dans une cellule cible permet de contrôler la transcription d'ARNm cellulaires. De telles séquences peuvent par exemple être transcrites, dans la cellule cible, en ARN complémentaires des ARNm cellulaires Grb2 ou Grb3-3 et bloquer ainsi leur traduction en protéine, selon la technique décrite dans le brevet EP 140 308. De telles séquences peuvent être constituées par tout ou partie de la séquence nucléiques SEQ ID n° 1, transcrites dans l'orientation inverse.

Comme indiqué ci-avant, Grb2 est une protéine au moins bi-fonctionnelle, s'ancrant par son domaine SH2 à des séquences particulières phosphorylées en tyrosine, et, par ses deux domaines SH3, aux facteurs d'échange de la famille SOS. Grb3-3 ayant perdu sa capacité à s'associer à des protéines phosphorylées en tyrosine peut donc uniquement former un complexe avec les protéines SOS. Grb3-3 peut donc s'opposer au recrutement du complexe Grb2-SOS par les récepteurs des facteurs de croissance autophosphorylés ou par des protéines associées également phosphorylées en tyrosine telles que SHC ou IRS1. Grb3-3 étant capable de bloquer ce recrutement, il est capable de bloquer des voies mitogènes et d'induire la mort cellulaire. La demanderesse a en effet démontré que la protéine Grb3-3 était exprimée au cours de certains processus physiologiques comme par exemple la maturation du thymus chez le rat. La demanderesse a également montré que Grb3-3 est capable d'induire la mort cellulaire par apoptose de différents types cellulaires. Ces propriétés tout à fait avantageuses ont pu être mises en évidence (i) par injection de la proréine recombinante dans les fibroblastes 3T3 et (ii) par par transfert de la séquence codant pour Grb3-3 dans les cellules 3T3 (exemple 4). Grb3-3 est donc capable d'induire la mort cellulaire de cellules viables telles que des cellules immortalisées, cancéreuses ou embryonnaires. Comme montré dans les exemples, Grb2 est capable de s'opposer aux effets de Grb3-3.

Par ailleurs, une recherche de l'expression de Grb3-3 réalisée au cours de l'infection de cellules lymphocytaires par le virus HIV a permis de montrer que la production virale massive observée 7 jours après l'infection est corrélée avec une surexpression de l'ARNm de Grb3-3 par les cellules infectées (exemple 5). Cette expérience montre que éliminer ou contrecarrer les effets cellulaires de Grb3-3 peut également permettre de maintenir en vie des cellules infectées, notamment par le VIH, et ainsi, permettre aux lymphocytes T4 de continuer à remplir un rôle de défense immunitaire. A cet égard, l'invention concerne également l'utilisation de composés capables d'éliminer ou de contrecarrer au moins partiellement les effets cellulaires de Grb3-3 pour la préparation d'une composition pharmaceutique destinée traitement du Sida. Plus particulièrement, les composés utilisés peuvent être :
- des séquences antisens génétiques telles que définies ci-dessus,
- des oligonucléotides spécifiques de Grb3-3, modifiés ou non pour une meilleure stabilité ou biodisponibilité (phosphorothioates, agents intercalants, etc). Il peut s'agir de préférence d'oligonucléotides recouvrant la séquence codante localisée entre le domaine SH3 N-terminal et le domaine SH2 résiduel.
- toute séquence dont le transfert dans les cellules infectées induit une surexpression de Grb2.

Les séquences nucléiques selon l'invention peuvent être utilisées telles quelles, par exemple après injection à l'homme ou l'animal, pour induire une protection ou traiter les cancers. En particulier, elles peuvent être injectées sous forme d'ADN nu selon la technique décrite dans la demande WO 90/11092. Elles peuvent également être administrées sous forme complexée, par exemple avec du DEAE-dextran (Pagano et al., J.Virol. 1 (1967) 891), avec des protéines nucléaires (Kaneda et al., Science 243 (1989) 375), avec des lipides (Felgner et al., PNAS 84 (1987) 7413), sous forme de liposomes (Fraley et al., J.Biol.Chem. 255 (1980) 10431), etc.

Préférentiellement, les séquences nucléiques selon l'invention font partie d'un vecteur. L'emploi d'un tel vecteur permet en effet d'améliorer l'administration de l'acide nucléique dans les cellules à traiter, et également d'augmenter sa stabilité dans lesdites cellules, ce qui permet d'obtenir un effet thérapeutique durable. De plus, il est possible d'introduire plusieurs séquences d'acide nucléique dans un même vecteur, ce qui augmente également l'efficacité du traitement.

Le vecteur utilisé peut être d'origine diverse, dès lors qu'il est capable de transformer les cellules animales, de préférence les cellules tumorales humaines. Dans un mode préféré de mise en oeuvre de l'invention, on utilise un vecteur viral, qui peut être choisi parmi les adénovirus, les rétrovirus, les virus adéno-associés (AAV), le virus de l'herpès, le cytomégalovirus (CMV), le virus de la vaccine, etc. Des vecteurs dérivés des adénovirus, des rétrovirus, ou des AAV incorporant des séquences d'acides nucléiques hétérologues ont été décrits dans la littérature [Akli et al., Nature Genetics 3 (1993) 224 ; Stratford-Perricaudet et al., Human Gene Therapy 1 (1990) 241 ; EP 185 573, Levrero et al., Gene 101 (1991) 195 ; Le Gal la Salle et al., Science 259 (1993) 988 ; Roemer et Friedmann, Eur. J. Biochem. 208 (1992) 211 ; Dobson et al., Neuron 5 (1990) 353 ; Chiocca et al., New Biol. 2 (1990) 739 ; Miyanohara et al., New Biol. 4 (1992) 238 ; WO91/18088].

La présente invention concerne donc également tout virus recombinant comprenant, insérée dans son génome, une séquence nucléique telle que définie avant.

Avantageusement, le virus recombinant selon l'invention est un virus défectif Le terme "virus défectif" désigne un virus incapable de se répliquer dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par l'acide nucléique de l'invention. Préférentiellement, le virus défectif conserve néanmoins les séquences de son génome qui sont nécessaires à l'encapsidation des particules virales.

Il est particulièrement avantageux d'utiliser les séquences nucléiques de l'invention sous forme incorporée à un adénovirus, un AAV ou un rétrovirus recombinant défectif.

Concernant les adénovirus, il en existe différents sérotypes, dont la structure et les propriétés varient quelque peu, mais qui ne sont pas pathogènes pour l'homme, et notamment les sujets non immuno-déprimés. Par ailleurs, ces virus ne s'intègrent pas dans le génome des cellules qu'ils infectent, et peuvent incorporer des fragments importants d'ADN exogène. Parmi les différents sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B.

Les virus recombinants défectifs de l'invention peuvent être préparés par recombinaison homologue entre un virus défectif et un plasmide portant entre autre la séquence nucléotidique telle que définie ci-avant (Levrero et al., Gene 101 (1991) 195; Graham, EMBO J. 3(12) (1984) 2917). La recombinaison homologue se produit après co-transfection desdits virus et plasmide dans une lignée cellulaire appropriée. La lignée cellulaire utilisée doit de préférence (i) être transformable par lesdits éléments, et (ii), comporter les séquences capables de complémenter la partie du génome du virus défectif, de préférence sous forme intégrée pour éviter les risques de recombinaison. A titre d'exemple de lignée utilisable pour la préparation d'adénovirus recombinants défectifs, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %). A titre d'exemple de lignée utilisable pour la préparation de rétrovirus recombinants défectifs, on peut mentionner la lignée CRIP (Danos et Mulligan, PNAS 85 (1988) 6460).

Ensuite, les virus qui se sont multipliés sont récupérés et purifiés selon les techniques classiques de biologie moléculaire.

La présente invention a également pour objet une composition pharmaceutique comprenant au moins un virus recombinant ou une séquence nucléotidique tels que définis ci-dessus.

Les compositions pharmaceutiques de l'invention peuvent être formulées en vue d'une administration par voie topique, orale, parentérale, intranasale, intraveineuse, intramusculaire, sous-cutanée, intraoculaire, etc.

Préférentiellement, les compositions pharmaceutiques contiennent des véhicules pharmaceutiquement acceptables pour une formulation injectable, éventuellement directement dans la tumeur à traiter. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses d'acides nucléiques (séquence ou vecteur) utilisées pour l'administration peuvent être adaptées en fonction de différents paramètres, et notamment en fonction du mode d'administration utilisé, de la pathologie concernée, de l'acide nucléique à exprimer, ou encore de la durée du traitement recherchée. D'une manière générale, concernant les virus recombinants selon l'invention, ceux-ci sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

De telles compositions pharmaceutiques peuvent être utilisées chez l'homme, pour le traitement et/ou la prévention des cancers. En particulier, les produits de l'invention étant capables de moduler l'activité des protéines ras, ils permettent d'intervenir dans le processus de développement des cancers, et notamment, ils peuvent inhiber l'activité des oncogènes dont l'activité transformante passe par une interaction p21-GAP fonctionnelle. De nombreux cancers ont en effet été associés à la présence de protéines ras oncogéniques. Parmi les cancers renfermant le plus souvent des gènes ras mutés, on peut citer notamment les adénocarcinomes du pancréas, dont 90 % ont un oncogène Ki-ras muté sur le douzième codon (Almoguera et coll., Cell 53 (1988) 549), les adénocarcinomes du colon et les cancers de la thyroïde (50 %), ou les carcinomes du poumon et les leucémies myéloïdes (30 %, Bos, J.L. Cancer Res. 49 (1989) 4682). Plus généralement, les compositions selon l'invention peuvent être utilisées pour traiter tout type de pathologie dans lesquelles un prolifèration cellulaire anormale est observée, par induction de l'apoptose, ainsi que toute pathologie caractérisée par une mort cellulaire par apoptose (sida, chorée de Huntington, Parkinson), au moyen de composés bloquant les effets de Grb3-3 (antisens notamment).

La présente invention sera plus complètement décrite à l'aide des exemples qui suivent, qui doivent être considérés comme illustratifs et non limitatifs.

### Légende des Figures

Figure 1 : Représentation schématique des domaines structuraux de Grb2 et Grb3-3.
Figure 2 : Etude de la liaison de Grb3-3 au récepteur à l'EGF (figure 2a) et à des peptides riches en proline (figure 2b).
Figure 3 : Effet de Grb3-3 sur la transactivation par ras d'un RRE dérivé de l'enhancer du virus du polyome.
Figure 4 : Mise en évidence de la mort cellulaire induite par Grb3-3 sur les fibroblastes 3T3.
Figure 5 : Mise en évidence de l'expression de Grb3-3 dans les cellules infectées par le virus HIV.

### Techniques Générales de Biologie Moléculaire

Les méthodes classiquement utilisées en biologie moléculaire telles que les extractions préparatives d'ADN plasmidique, la centrifugation d'ADN plasmidique en gradient de chlorure de césium, l'électropborèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982 ; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### EXEMPLES

### 1. Isolement du gène Grb3-3

Le gène Grb3-3 a été isolé par criblage d'une banque d'ADN humain au moyen d'une sonde dérivée de la séquence du gène Grb2.

500 000 phages recombinants Lambda gt11 portant des fragments d'ADN issus d'une banque de placenta humaine (Clontech) ont été criblés au moyen d'une sonde dérivée de la séquence du gène Grb2. La sonde utilisée correspond aux 8 premiers acides aminés de la protéine Grb2, et possède la séquence suivante:
ATGGAAGCCATCGCCAAATATGAC (SEQ ID n° 2)

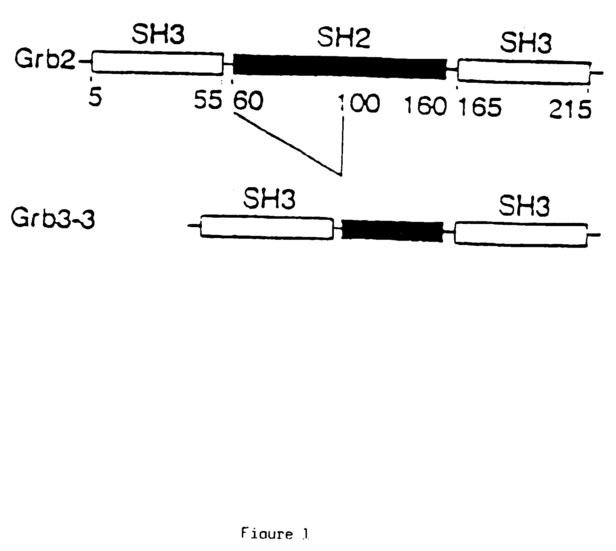

10 clones positifs ont ainsi été identifiés. L'insert de ces 10 clones a été isolé sous forme de fragments EcoRI, cloné dans le plasmide M13mp18 et séquencé. Parmi ces 10 clones, 9 portaient des inserts identiques à la séquence Grb2. Un seul d'entreeux portait un insert d'une taille inférieure au gène Grb2, en raison d'une délétion dans le domaine SH2 (Figure 1). L'analyse de la séquence restante a révélé une identité parfaite avec les régions correspondantes de Grb2, y compris dans les régions 5' et 3' non codantes. La phase de lecture ouverte de ce clone code pour une protéine de 177 acides aminés (SEQ ID n° 1), comprenant 2 domaines SH3 bordant un domaine SH2 incomplet (figure 1). Les acides aminés délétés dans le domaine SH2 (résidus 60 à 100 de la protéine Grb2) correspondent aux résidus impliqués dans la liaison de Grb2 aux peptides contenant des tyrosines phosphorylées.

### 2. Activité de liaison de la protéine Grb3-3

Comme indiqué plus haut, la protéine Grb2 est le médiateur de l'interaction entre les récepteurs de facteurs de croissance phosphorylés et les facteurs SOS. Cet exemple démontre que la protéine Grb3-3 est incapable d'interagir avec le récepteur à l'EGF phosphorylé, mais qu'elle conserve sa faculté d'interagir avec un peptide riche en proline dérivé de la séquence du facteur SOS1 humain.

La capacité de liaison de Grb3-3 a été étudiée en utilisant des protéines de fusion à la Glutathion-S-Transférase (GST), biotinylées. Ce type de fusion permet une purification rapide et efficace des produits recombinants. Pour cela, les séquences de l'invention ont été exprimées dans la souche d'*E.coli* TG1 sous forme de protéines de fusion avec la GST selon la technique décrite par Smith et Johnson [Gene 67 (1988) 31]. Brièvement, les gènes Grb2 et Grb3-3 ont tout d'abord été modifiés par introduction de part et d'autre des codons start et stop d'un site BamHI. Pour cela, les phases ouvertes de lecture de ces gènes ont été amplifiées par PCR au moyen des oligonucléotides suivants :
Oligonucléotide I (5') (SEQ ID n° 3):
   GAATTCGGATCCATGGAAGCCATCGCCAAATATGACTTC
Oligonucléotide II (3') (SEQ ID n° 4) :
   GAATTCGGATCCTTAGACGTTCCGGTTCACGGGGGTGAC

La partie soulignée correspond au site BamHI crée, suivi ou précédé des codons start et stop.

Les gènes ainsi amplifiés ont ensuite été clonés sous forme de fragments BamHI dans le vecteur pGEX 2T (Pharmacia) linéarisé par le même enzyme, en 3' et en phase d'un ADNc codant pour la GST. Les vecteurs ainsi obtenus ont ensuite été utilisés pour transformer la souche *E.coli* TG1. Les cellules ainsi transformées ont été précultivées une nuit à 37°C, diluées au 1/10e dans du milieu LB, ajoutées d'IPTG pour induire l'expression (2 heures, 25°C), puis cultivées 21 heures environ à 25°C. Les cellules ont ensuite été lysées, et les protéines de fusions produites purifiées par affinité sur colonne Agarose-GSH. Pour cela, le lysat bactérien est incubé en présence du gel (préparé et équilibré avec le tampon de lyse) pendant 15 minutes à 4°C. Après 3 lavages avec un tampon Tris-HCl pH 7,4, les protéines sont éluées en présence d'un tampon Tris-HCl pH 7,7 contenant un excès de GST. Le surnageant est récolté et centrifugé.

Le même protocole a été utilisé pour préparer un mutant de Grb2 dans lequel la glycine 203 est remplacée par une arginine (Grb2G203R) et un mutant de Grb3-3 dans lequel la glycine 162 est remplacée par une arginine (Grb3-3G162R). Le mutant Grb2G203R a été décrit comme n'ayant plus d'activité dans un test de réinitiation de la synthèse d'ADN (Lowenstein et ai précitée). Le mutant Grb3-3G162R porte la même mutation sur la même position, et devrait donc également être inactif

Ces mutants ont été préparés par mutagénèse par PCR sur les gènes Grb2 et Grb3-3 en utilisant, en 5', l'oligonucléotide I décrit ci-dessus, et en 3', l'oligonucléotide III suivant sur lequel le codon muté est souligné :
Oligonucléotide III (3') (SEQ ID n° 5):
   GACGTTCCGGTTCACGGGGGTGACATAATTGCGGGGAAACATGCGGGTC

Les fragments ainsi amplifiés ont ensuite été élués, réamplifiés par PCR au moyen des oligonucléotides I et II, puis clonés dans le vecteur pGEX 2T. Les mutants ont ensuite été produits comme décrit ci-dessus.

Les protéines de fusion à la GST (GST-Grb2, GST-Grb3-3, GST-Grb3-3G162R et la GST) ont ensuite été biotinylées par les techniques classiques connues de l'homme du métier (Cf techniques générales de biologie moléculaire ainsi que Mayer et al., PNAS 88 (1991) 627), et utilisées comme sondes pour déterminer la liaison au récepteur à l'EGF phosphorylé immobilisé (2.1.) puis à un peptide dérivé de hSOS1 (2.2.).

### 2.1. Liaison au récepteur EGF phosphorylé

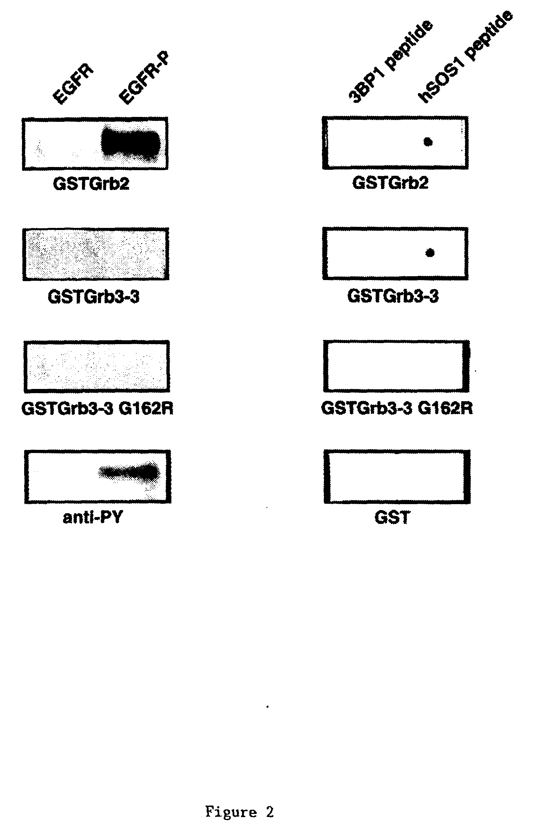

Protocole : Le récepteur à l'EGF utilisé a été purifié à partir de cellules A431 par immobilisation sur WGA-sépharose selon la technique décrite par Duchesne et al. (Science 259 (1993) 525). 2 µg de ce récepteur ont d'abord été stimulés par 1 µM EGF, 10 min. à 22°C, puis incubés, avec ou sans ATP froid (10 µM), en présence de 2,5 mM MnCl2 dans un tampon HNTG (20 mM Hepes, 150 mM NaCl, 0,1 % Triton, 10 % Glycérol, pH=7,5) à 4°C pendant 2 min. La phosphorylation du récepteur est ensuite stopée par addition d'un tampon de dégradation. Les échantillons sont ensuite déposés sur un gel SDS-PAGE 4-20 % puis transférés sur des membranes de polyvinylgdène difluorure (PVDF). Les blots ont ensuite été incubés en présence des différentes fusions GST biotinylées (2 µg/ml), puis révélés au moyen de streptavidine couplée à la phosphatase alkaline (Promega). Les récepteurs EGF ont également été soumis à un immunoblot en présence d'anticorps anti-phosphotyrosines (anti--PY) pour vérifier que les récepteurs ont bien été phosphorylés.
Résultats : Les résultats obtenus sont présentés sur la figure 2a. Ils montrent, comme attendu, que la protéine Grb2 interagit avec le récepteur EGF sous forme phosphorylée seulement. Ils montrent ensuite que la protéine Grb3-3 ne lie pas le récepteur EGF, quel que soit son degré de phosphorylation.

### 2.2. Liaison à un peptide dérivé de hSOS1

Protocole : Les deux peptides fiches en proline suivants ont été synthétisés :
Peptide hSOS1 : GTPEVPVPPPVPPRRRPESA : Ce peptide correspond aux résidus 1143 à 1162 de la protéine hSOS1 (Li et al., Nature 363 (1993) 83), responsable de l'interaction entre Grb2 et hSOS1 (SEQ ID n° 6).
Peptide 3BP1 : PPPLPPLV : Ce peptide est dérivé de la protéine 3BP1, qui est connue pour lier de manière efficace le domaine SH3 de Abl et Src (Cicchetti et al., Science 257 (1992) 803) (SEQ ID n° 7).
   Chacun de ces peptides (1 µl, 10 mg/ml) a été immobilisé sur membrane de nitrocellulose. Les membranes ont ensuire été incubées dans un tampon bloquant (Tris 20 mM pH=7,6, 150 mM NaCl, 0,1 % Tween, 3 % albumine bovine). Les membranes ont ensuite été incubées une nuit à 4°C en présence des différentes fusions GST biotinylées (4 µg/ml), puis révélées au moyen de streptavidine couplée à la phosphatase alkaline (Promega)
Résultats : Les résultats obtenus sont présentés sur la figure 2b. Ils montrent que Grb3-3, comme Grb2, est capable de lier le peptide hSOS1. Ils montrent de plus que cette interaction est spécifique puisqu'aucune liaison n'est observée avec le peptide 3BP1. Par ailleurs, les résultats montrent également que le mutant Grb3-3G162R n'est plus capable de lier le peptide hSOS1, ce qui confirme l'importance de ce résidu et le role fonctionnel de cette interaction.

### 3. Activité de la protéine Grb3-3

Cet exemple démontre que, en dépit de sa délétion dans le domaine SH2, la protéine Grb3-3 possède un effet fonctionnel.

L'activité de la protéine Grb3-3 a été étudié par détermination de sa capacité à coopérer avec ras pour la transactivation d'un promoteur possédant des éléments de réponse à ras (RRE), et gouvernant l'expression d'un gêne reporteur.

Le protocole utilisé a été décrit par exemple dans Schweighoffer et al., Science 256 (1992) 825. Brièvement, le promoteur utilisé est un promoteur synthétique composé du promoteur murin du gène de la thymidine kinase et de 4 éléments PEA1 répétés dérivés de l'enhancer du polyôme (Wasylyk et al., EMBO J. 7 (1988) 2475): promoteur Py-TK. Ce promoteur dirige l'expression du gène reporteur, en l'occurrence du gène bactérien de la chloramphénicol acétyl transférase (CAT) : vecteur Py-TK-CAT. Les vecteurs d'expression des gènes testés ont été construits par insertion desdits gènes, sous forme de fragments BamHI, au site BglII du plasmide pSV2. Ce site permet de placer les gènes sous controle du promoteur précoce SV40.

Des cellules ER22 à 40% de confluence ont été transfectées avec 0,5 µg du vecteur Py-TK-CAT seul (Py) ou en présence de du vecteur d'expression portant, sous contrôle du promoteur précoce de SV40, le gène : Grb2, 2 µg, Grb3-3, 2 µg, Grb2(G203R) 2 µg, Grb3-3(G162R) 2 µg, ou Grb3-3, 2 µg + Grb2, 2 µg. Dans chaque cas, la quantité totale d'ADN a été ajustée à 5 µg avec un vecteur d'expression sans insert. La transfection a été effectuée en présence de lipospermine (Transfectam, IBF-Sepracor). Les cellules ont été maintenues 48 heures en culture dans un milieu DMEM supplémenté par 0,5 % serum de veau fétal. L'activité CAT (transactivation du RRE) a ensuite été déterminée comme décrit par Wasylyk et al (PNAS 85 (1988) 7952).

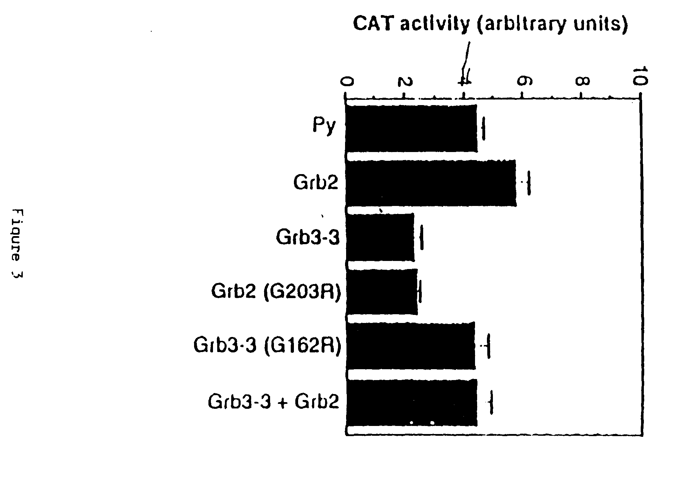

Les résultats obtenus sont présentés sur la figure 3. Ils montrent clairement que l'expression de la protéine Grb3-3 s'oppose aux effets de l'activation d'un récepteur à facteur de croissance. Ils montrent également que Grb2 en excès s'oppose aux effets de Grb3-3 sur la réponse au facteur de croissance.

### 4 Grb3-3 induit l'apoptose cellulaire

Cet exemple démontre l'implication directe de Grb3-3 dans l'apoptose cellulaire. Cette propriété offre des applications particulièrement avantageuses pour le traitement des pathologies résultant d'une prolifération cellulaire (cancers, resténose, etc).

L'induction de l'apoptose cellulaire par Grb3-3 a été démontrée (i) par injection de la protéine recombinante dans les fibroblastes 3T3 et (ii) par par transfert de la séquence codant pour Grb3-3 dans les cellules 3T3.

### (i) Injection de la protéine recombinante

La protéine Grb3-3 recombinante a été préparée sous forme de protéine de fusion avec la GST selon le protocole décrit dans l'exemple 2. La protéine de fusion a ensuite été traitée par la thrombine (0,25%, Sigma) pour séparer la partie GST, puis purifiée par chromatographie d'échange d'ions sur colone monoQ. Les fractions contenant la protéine recombinante ont ensuite été concentrées au moyen de microconcentreurs Microsep (Filtron) dans un tampon phosphate 20 mM (pH 7) contenant 100 mM NaCl. La protéine purifiée ainsi obtenue a été injectée (1 à 3 mg/ml) à des cellules 3T3 en culture au moyen d'un microinjecteur automatique Eppendorf. Les cellules ont ensuite été incubées à 34°C et photographiées à intervalles réguliers pour suivre les transformations morphologiques. Les résultats obtenus montrent que 5 heures après l'injection de Grb3-3 la majeure partie des cellules sont mortes alors que l'injection dans les mêmes conditions de Grb2 ou du mutant Grb3-3 (G162R) n'a aucun effet sur la viabilité des cellules.

### (ii) Transfert de la séquence codant pour la protéine recombinante

Un plasmide a été construit comprenant la séquence SEQ ID n° 1 codant pour la protéine Grb3-3 sous le controle du promotyeur précoce du virus SV40.

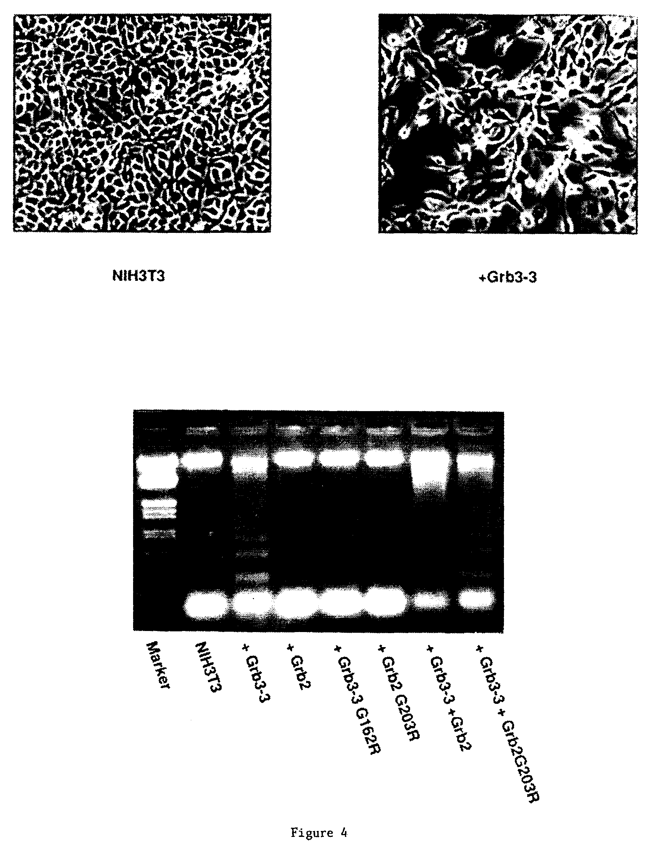

Les fibroblastes 3T3 à 40 % de confluence ont été transfectés en présence de lipospermine (Transfectam, IBF-Sepracor) avec 0,5 ou 2 µg de ce plasmide d'expression. 48 heures après la transfection, 50% des cellules étaient en suspension dans le milieu, et les cellules restantes, adhérant à la paroi, présentaient des changements morphologiques très importants (figure 4). Une analyse par électrophorèse en gel d'agarose a montré par ailleurs que les cellules présentaient un pattern de fragmentation d'ADN oligo-nucléosomal caractéristique des cellules mortes (figure 4). En revanche, les cellules transfectées dans les mêmes conditions par un plasmide d'expression de Grb2, Grb3-3 (G162R) ou Grb2 (G203R) conservent une morphologie normale, sont toujours viables et ne présentent aucune fragmentation d'ADN. Comme le montre la figure 4, la co-expression de Grb2 permet de s'opposer aux effets de Grb3-3.

Ces résultats montrent donc clairement que Grb3-3 constitue un gène tueur capable d'induire l'apoptose cellulaire. Comme indiqué ci-avant, cette propriété offre des applications particulièrement avantageuses pour le traitement des pathologies résultant d'une prolifération cellulaire telles que notamment les cancers, la resténose, etc.

### 5. Mise en évidence de l'expression de Grb3-3 dans les lymphocytes infectés par le virus HIV.

Cet exemple montre que, au cours du cycle d'infection des lymphocytes T par le virus HIV, la proportion relative des ARNm de Grb2 et Grb3-3 est modifiée, et que le messager de Grb3-3 est surexprimé au moment de la production virale massive et de la mort cellulaire.

Des lymphocytes du sang périphérique ont été infectés par le virus HIV-1 à deux dilutions (1/10 et 1/100) pendant 1, 4 ou 7 jours. Les ARNm des cellules ont ensuite été analysés par reverse-PCR au moyen d'oligonucléotides spécifiques de Grb2 et Grb3-3 pour déterminer la proportion relative des messagers de Grb2 et Grb3-3. Les oligonucléotides spécifiques de Grb3-3 utilisés sont les suivants :
Oligonucléotide IV (3') : ATCGTTTCCAAACGGATGTGGTTT (SEQ ID n° 8)
Oligonucléotide V (5') : ATAGAAATGAAACCACATCCGTTT (SEQ ID n° 9)

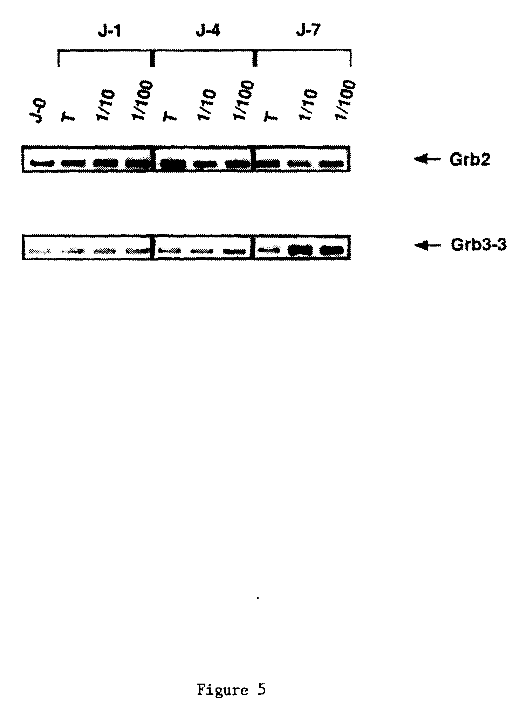

Les résultats obtenus sont présentés sur la figure 5. Ils montrent clairement que 7 jours après l'infection par le virus HIV, l'ARNm de Grb3-3 est surexprimé. Comme montré par dosage de la protéine p24 et de la transcriptase inverse du virus, le jour 7 correspond également à la période à laquelle une production virale massive est observée.

## Revendications

1. Séquence nucléotidique représentée en séquence SEQ ID n° 1.

2. Séquence nucléotidique représentée en séquence SEQ ID n° 1 et capable d'inhiber au moins en partie l'expression de la protéine Grb2 ou Grb3-3.

3. Polypeptide représenté en séquence SEQ ID n°1.

4. Vecteur comprenant une séquence nucléotidique selon les revendications 1 ou 2.

5. Vecteur selon la revendication 4 caractérisé en ce qu'il s'agit d'un vecteur viral.

6. Vecteur selon la revendication 5 caractérisé en ce qu'il s'agit d'un vecteur dérivé des adénovirus, des rétrovirus, des AAV, du virus HSV, CMV ou du virus de la vaccine.

7. Vecteur selon les revendications 5 ou 6 caractérisé en ce qu'il s'agit d'un virus défectif pour la réplication.

8. Composition pharmaceutique comprenant un ou plusieurs vecteurs selon l'une des revendications 4 à 7.

9. Composition pharmaceutique comprenant une ou plusieurs séquences nucléotidiques selon les revendications 1 ou 2, sous forme complexée à du DEAE-dextran, à des protéines nucléaires ou à des lipides, sous forme brute ou encore incorporée à des liposomes.

10. Utilisation d'une séquence selon la revendication 1 ou 2 ou d'un vecteur contenant ladite séquence pour la préparation d'une composition pharmaceutique destinée au traitement des cancers.

11. Utilisation d'un composé capable d'éliminer ou de contrecarrer au moins partiellement les effets cellulaires de Grb3-3 pour la préparation d'une composition pharmaceutique destinée traitement du Sida.

## Claims

1. Nucleotide sequence represented in sequence SEQ ID No. 1.

2. Nucleotide sequence represented in sequence SEQ ID No. 1 and capable of inhibiting, at least partially, the expression of the Grb2 or Grb3-3 protein.

3. Polypeptide represented in sequence SEQ ID No. 1.

4. Vector comprising a nucleotide sequence according to Claims 1 or 2.

5. Vector according to Claim 4, characterized in that it is a viral vector.

6. Vector according to Claim 5, characterized in that it is a vector derived from adenoviruses, retroviruses, AAVs, HSV virus, CMV or vaccinia Virus.

7. Vector according to Claim 5 or 6, characterized in that it is a virus defective for replication.

8. Pharmaceutical composition comprising one or more vectors according to one of Claims 4 to 7.

9. Pharmaceutical composition comprising one or more nucleotide sequences according to Claims 1 or 2, in a form complexed with DEAE-dextran, with nuclear proteins or with lipids, in crude form or alternatively incorporated into liposomes.

10. Use of a sequence according to Claim 1 or 2 or of a vector containing the said sequence for the preparation of a pharmaceutical composition intended for the treatment of cancers.

11. Use of a compound capable of eliminating or blocking, at least partially, the cellular effects of Grb3-3 for the preparation of a pharmaceutical composition intended for the treatment of AIDS.

## Patentansprüche

1. Nucleotidsequenz der Sequenz SEQ ID NO. 1.

2. Nucleotidsequenz der Sequenz SEQ ID NO. 1 mit der Fähigkeit, mindestens teilweise die Expression des Proteins Grb2 oder Grb3-3 zu hemmen.

3. Polypeptid der Sequenz SEQ ID NO. 1.

4. Vektor, umfassend eine Nucleotidsequenz nach einem der Ansprüche 1 oder 2.

5. Vektor nach Anspruch 4, dadurch gekennzeichnet, daß er ein viraler Vektor ist.

6. Vektor nach Anspruch 5, dadurch gekennzeichnet, daß er ein von Adenoviren, Retroviren, AAV, HSV-, CMV-Virus oder Impfviren abgeleiteter Vektor ist.

7. Vektor nach den Ansprüchen 5 oder 6, dadurch gekennzeichnet, daß er ein replikationsdefektes Virus ist.

8. Pharmazeutische Zusammensetzung, umfassend einen oder mehrere Vektor(en) nach einem der Ansprüche 4 bis 7.

9. Pharmazeutische Zusammensetzung, umfassend eine oder mehrere Nucleotidsequenz(en) nach den Ansprüchen 1 oder 2 in mit DEAE-Dextran, Kernproteinen oder Lipiden komplexierter Form, in roher Form oder auch eingebaut in Liposomen.

10. Verwendung einer Sequenz nach Anspruch 1 oder 2 oder eines diese Sequenz enthaltenden Vektors zur Herstellung einer pharmazeutischen Zusammensetzung zur Behandlung von Krebs.

11. Verwendung einer Verbindung mit der Fähigkeit zur Beseitigung der oder zur mindestens teilweisen Wirkung gegen die zellulären Wirkungen von Grb3-3 zur Herstellung einer pharmazeutischen Zusammensetzung zur Behandlung von AIDS.


LISTE DE SEQUENCES

(1) INFORMATION GENERALE:

(i) DEPOSANT:
(A) NOM: RHONE-POULENC RORER S.A.
(B) RUE: 20, avenue Raymond ARON
(C) VILLE: ANTONY
(E) PAYS: FRANCE
(F) CODE POSTAL: 92165

(ii) TITRE DE L' INVENTION: Gene Grb3-3, ses variants et leurs
utilisations.

(iii) NOMBRE DE SEQUENCES: 9

(iv) FORME LISIBLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)

(2) INFORMATION POUR LA SEQ ID NO: 1:

(i) CARACTERISTIQUES DE LA SEQUENCE:
(A) LONGUEUR: 933 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(vi) ORIGINE:
(A) ORGANISME: Homo sapiens

(ix) CARACTERISTIQUE ADDITIONELLE:
(A) NOM/CLE: CDS
(B) EMPLACEMENT: 37..567
(D) AUTRES RENSEIGNEMENTS: /product= "Grb3-3"

(xi) DESCRIPTION DE LA SEQUENCE: SEQ ID NO: 1:

GAATTCGGGG CTGCTGAGCA CTGAGCAGGG CTCAGA ATG GAA GCC ATC GCC AAA 54
Met Glu Ala Ile Ala Lys
1               5

TAT GAC TTC AAA GCT ACT GCA GAC GAC GAC CTG AGC TTC AAA AGG GGG 102
Tyr Asp Phe Lys Ala Thr Ala Asp Asp Asp Leu Ser Phe Lys Arg Gly
            10                  15                  20

GAC ATC CTC AAG GTT TTG AAC GAA GAA TGT GAT CAG AAC TGG TAC AAG 150
Asp Ile Leu Lys Val Leu Asn Glu Glu Cys Asp Gln Asn Trp Tyr Lys
        25                  30                  35

GCA GAG CTT AAT GGA AAA GAC GGC TTC ATT CCC AAG AAC TAC ATA GAA 198
Ala Glu Leu Asn Gly Lys Asp Gly Phe Ile Pro Lys Asn Tyr Ile Glu

40                    45                    50

ATG AAA CCA CAT CCG TTT GGA AAC GAT GTG CAG CAC TTC AAG GTG CTC     246
Met Lys Pro His Pro Phe Gly Asn Asp Val Gln His Phe Lys Val Leu
 55                  60                  65                  70

CGA GAT GGA GCC GGG AAG TAC TTC CTC TGG GTG GTG AAG TTC AAT TCT     294
Arg Asp Gly Ala Gly Lys Tyr Phe Leu Trp Val Val Lys Phe Asn Ser
                 75                  80                  85

TTG AAT GAG CTG GTG GAT TAT CAC AGA TCT ACA TCT GTC TCC AGA AAC     342
Leu Asn Glu Leu Val Asp Tyr His Arg Ser Thr Ser Val Ser Arg Asn
             90                  95                 100

CAG CAG ATA TTC CTG CGG GAC ATA GAA CAG GTG CCA CAG CAG CCG ACA     390
Gln Gln Ile Phe Leu Arg Asp Ile Glu Gln Val Pro Gln Gln Pro Thr
        105                 110                 115

TAC GTC CAG GCC CTC TTT GAC TTT GAT CCC CAG GAG GAT GGA GAG CTG     438
Tyr Val Gln Ala Leu Phe Asp Phe Asp Pro Gln Glu Asp Gly Glu Leu
    120                 125                 130

GGC TTC CGC CGG GGA GAT TTT ATC CAT GTC ATG GAT AAC TCA GAC CCC     486
Gly Phe Arg Arg Gly Asp Phe Ile His Val Met Asp Asn Ser Asp Pro
135                 140                 145                 150

AAC TGG TGG AAA GGA GCT TGC CAC GGG CAG ACC GGC ATG TTT CCC CGC     534
Asn Trp Trp Lys Gly Ala Cys His Gly Gln Thr Gly Met Phe Pro Arg
                155                 160                 165

AAT TAT GTC ACC CCC GTG AAC CGG AAC GTC TAAGAGTCAA GAAGCAATTA       584
Asn Tyr Val Thr Pro Val Asn Arg Asn Val
            170                 175

TTTAAAGAAA GTGAAAAATG TAAAACACAT ACAAAAGAAT TAAACCCACA AGCTGCCTCT   644

GACAGCAGCC TGTGAGGGAG TGCAGAACAC CTGCCGGGTC ACCCTGTGAC CCTCTCACTT   704

TGGTTGGAAC TTTAGGGGGT GGGAGGGGGC GTTGGATTTA AAAATGCCAA AACTTACCTA   764

TAAATTAAGA AGAGTTTTTA TTACAAATTT TCACTGCTGC TCCTCTTTCC CCTCCTTTGT   824

CTTTTTTTTC TTCCTTTTTT CTCTTCTGTC CATCAGTGCA TGACGTTTAA GGCCACGTAT   884

AGTCCTAGCT GACGCCAATA AAAAACAAGA AACCAAAAAA CCCGAATTC               933

(2) INFORMATION POUR LA SEQ ID NO: 2:

    (i) CARACTERISTIQUES DE LA SEQUENCE:
        (A) LONGUEUR: 24 paires de bases
        (B) TYPE: acide nucléique
        (C) NOMBRE DE BRINS: simple
        (D) CONFIGURATION: linéaire

   (ii) TYPE DE MOLECULE: ADNc
   (vi) ORIGINE:
        (A) ORGANISME: OLIGONUCLEOTIDE

   (xi) DESCRIPTION DE LA SEQUENCE: SEQ ID NO: 2:

ATGGAAGCCA TCGCCAAATA TGAC                                          24

(2) INFORMATION POUR LA SEQ ID NO: 3:

(i) CARACTERISTIQUES DE LA SEQUENCE:
(A) LONGUEUR: 39 paires de bases
(B) TYPE: acide nucléique
(C) NOMBRE DE BRINS: simple
(D) CONFIGURATION: linéaire

(ii) TYPE DE MOLECULE: ADNc
(vi) ORIGINE:
(A) ORGANISME: OLIGONUCLEOTIDE I

(xi) DESCRIPTION DE LA SEQUENCE: SEQ ID NO: 3:

GAATTCGGAT CCATGGAAGC CATCGCCAAA TATGACTTC 39

(2) INFORMATION POUR LA SEQ ID NO: 4:

(i) CARACTERISTIQUES DE LA SEQUENCE:
(A) LONGUEUR: 39 paires de bases
(B) TYPE: acide nucléique
(C) NOMBRE DE BRINS: simple
(D) CONFIGURATION: linéaire

(ii) TYPE DE MOLECULE: ADNc
(vi) ORIGINE:
(A) ORGANISME: OLIGONUCLEOTIDE II

(xi) DESCRIPTION DE LA SEQUENCE: SEQ ID NO: 4:

GAATTCGGAT CCTTAGACGT TCCGGTTCAC GGGGGTGAC 39

(2) INFORMATION POUR LA SEQ ID NO: 5:

(i) CARACTERISTIQUES DE LA SEQUENCE:
(A) LONGUEUR: 49 paires de bases
(B) TYPE: acide nucléique
(C) NOMBRE DE BRINS: simple
(D) CONFIGURATION: linéaire

(ii) TYPE DE MOLECULE: ADNc
(vi) ORIGINE:
(A) ORGANISME: OLIGONUCLEOTIDE III

(xi) [?][?]SCRIPTION DE LA SEQUENCE: SEQ ID NO: 5:

GACGTTCCGG TTCACGGGGG TGACATAATT GCGGGGAAAC ATGCGGGTC 49

(2) INFORMATION POUR LA SEQ ID NO: 6:

(i) CARACTERISTIQUES DE LA SEQUENCE:
(A) LONGUEUR: 20 acides aminés
(B) TYPE: acide aminé
(D) CONFIGURATION: linéaire

(ii) TYPE DE MOLECULE: Protéine
(vi) ORIGINE:
(A) ORGANISME: Peptide hSOS1 (résidus 1143 à 1162)

(xi) DESCRIPTION DE LA SEQUENCE: SEQ ID NO: 6:

Gly Thr Pro Glu Val Pro Val Pro Pro Pro Val Pro Pro Arg Arg Arg

1               5                   10                  15

Pro Glu Ser Ala

            20

(2) INFORMATION POUR LA SEQ ID NO: 7:

(i) CARACTERISTIQUES DE LA SEQUENCE:
(A) LONGUEUR: 8 acides aminés
(B) TYPE: acide aminé
(D) CONFIGURATION: linéaire

(ii) TYPE DE MOLECULE: Protéine

(vi) ORIGINE:
(A) ORGANISME: Peptide 3BP1

(xi) DESCRIPTION DE LA SEQUENCE: SEQ ID NO: 7:

Pro Pro Pro Leu Pro Pro Leu Val

1               5

(2) INFORMATION POUR LA SEQ ID NO: 8:

(i) CARACTERISTIQUES DE LA SEQUENCE:
(A) LONGUEUR: 24 paires de bases
(B) TYPE: acide nucléique
(C) NOMBRE DE BRINS: simple
(D) CONFIGURATION: linéaire

(ii) TYPE DE MOLECULE: ADNc
(vi) ORIGINE:
(A) ORGANISME: OLIGONUCLEOTIDE IV

(xi) DESCRIPTION DE LA SEQUENCE: SEQ ID NO: 8:

ATCGTTTCCA AACGGATGTG GTTT                                        24

(2) INFORMATION POUR LA SEQ ID NO: 9:

(i) CARACTERISTIQUES DE LA SEQUENCE:
(A) LONGUEUR: 24 paires de bases
(B) TYPE: acide nucléique
(C) NOMBRE DE BRINS: simple
(D) CONFIGURATION: linéaire

(ii) TYPE DE MOLECULE: ADNc
(vi) ORIGINE:
(A) ORGANISME: OLIGONUCLEOTIDE V

(xi) DESCRIPTION DE LA SEQUENCE: SEQ ID NO: 9:
ATAGAAATGA AACCACATCC GTTT                                        24